(19) Europäisches Patentamt
European Patent Office
Office européen des brevets

(11) Numéro de publication: **0 246 171**
**A2**

# (12) DEMANDE DE BREVET EUROPEEN

(21) Numéro de dépôt: **87420126.2**

(22) Date de dépôt: **14.05.87**

(51) Int. Cl.4: **C 07 D 213/84**
C 07 F 7/08, A 01 N 43/40

(30) Priorité: **16.05.86 FR 8607260**

(43) Date de publication de la demande:
**19.11.87 Bulletin 87/47**

(84) Etats contractants désignés:
**AT BE CH DE ES FR GB GR IT LI LU NL SE**

(71) Demandeur: **RHONE-POULENC AGROCHIMIE**
**14-20, rue Pierre Baizet**
**F-69009 Lyon (FR)**

(72) Inventeur: **Lambert, Claude**
**12 Chemin de Montpellas**
**F-69009 Lyon (FR)**

**Pepin, Régis**
**27 Montée Castellane**
**F-69140 Rilleux la Pape (FR)**

(74) Mandataire: **Chrétien, François et al**
**RHONE-POULENC AGROCHIMIE BP 9163**
**F-69263 Lyon Cedex 09 (FR)**

(54) **Compositions fongicides à base de dérivés nicotiniques, nouveaux dérivés nicotiniques et leur préparation.**

(57) - Dérivés nicotiniques - Ils sont de formule

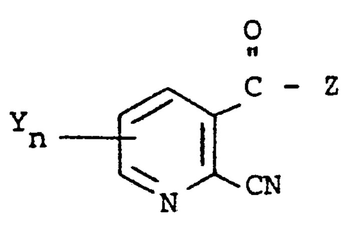

avec : Z = OR avec R = alcoyle ($C_1$-18C), alcényle (2-18C)
alcynyl (2-5C), phényle, phénoxy,
éventuellement substitués
Y = H,hal,alcoyle (1-4c) alcoxy (1-4c)
n = 1 à 3
-Produits fongicides pour l'agriculture.

EP 0 246 171 A2

**Description**

COMPOSITIONS FONGICIDES A BASE DE DERIVES NICOTINIQUES, NOUVEAUX DERIVES NICOTINIQUES ET LEUR PREPARATION

La présente invention concerne des compositions fongicides à base de dérivés nicotiniques, des dérivés nicotiniques nouveaux et l'utilisation de dérivés nicotiniques pour la lutte contre les maladies fongiques des plantes.

L'intention a plus particulièrement pour objet des compositions fongicides caractérisées en ce qu'elles contiennent comme matière active au moins un composé de formule :

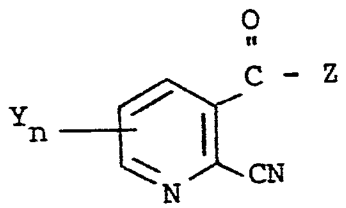

(I)

dans laquelle
- Z est un radical OR dans lequel
R est
• un atome d'hydrogène, un cation M/minéral, métallique ou ammonium, ou organique notamment ammonium substitué en particulier mono-, di-, ou trisubstitué par un radical alcoyle linéaire ou ramifié, de 1 à 18 aktomes de carbone, cycloalcoyle de 3 à 6 atomes de carbone ou alcanol de 1 à 3 atomes de carbone, ou
• un radical alcoyle, linéaire ou ramifié, de 1 à 18 atomes de carbone, ou alcényle de 2 à 18 atomes de carbone, alcynyle de 2 à 5 atomes de carbone chacun de ces radicaux pouvant être substitué par au moins un substituant choisi dans le groupe comprenant :
- un atome d'halogène, un groupe cyano,
- un radical alcoxy ou alcoylthio de 1 à 4 atomes de carbone,
- un radical phénoxy éventuellement substitué par au moins un substituant choisi dans le groupe comprenant un atome d'halogène, un alcoyle de 1 à 4 atomes de carbone ou un alcoxy de 1 à 4 atomes de carbone,
- un groupe COOR'ou OCOR', dans lequel R' est un alcoyle de 1 à 4 atomes de carbone, un alcényle de 2 à 4 atomes de carbone ou un phényle chacun des radicaux alcoyle,alcényle ou phényle étant éventuellement substitué par au moins un substituant choisi dans le groupe comprenant un atome d'halogène, un alcoyle de 1 à 4 atomes de carbone ou un alcoxy de 1 à 4 atomes de carbone,, un groupe I' de formule :

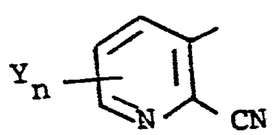

(I')

dans laquelle Y et n sont comme ci-après,
- ou un groupe phényle ou naphtyle éventuellement substitué par au moins un substituant choisi dans le groupe comprenant un atome d'halogène, un alcoyle de 1 à 4 atomes de carbone, un alcoxy de 1 à 4 atomes de carbone et le radical $NO_2$
- ou un groupe amino éventuellement substitué,
• ou un groupe phényle éventuellement substitué par au moins un substituant choisi dans le groupe comprenant un atome d'halogène, un alcoyle de 1 à 4 atomes de carbone et un alcoxy de 1 à 4 atomes de carbone
• ou pyridinyl alcoyl (de 1 à 3 atomes de carbone) éventuellement substitué par au moins 1 atome d'halogène et/ou un alcoyle de 1 à 4 atomes de carbone et/ou un groupe cyano,
• ou alcoyl- ou phényl- ou phénylalcoyl carbonylalcoyle, dont chaque partie alcoyle comprend 1 à 3 atomes de carbone et dont la partie phényle est éventuellement substituée par 1 à 5 substituants choisis dans le groupe comprenant un atome d'halogène, un alcoyle de 1 à 4 atomes de carbone, un alcoxy de 1 à 4 atomes de carbone et le radical $NO_2$
• ou un groupe tri alcoyl ($C_1$-$C_4$ silicyl alcoyle ($C_1$-$C_4$), la partie alcoyle pouvant être substituée par un phényle éventuellement substitué par un alcoyle de 1 à 4 atomes de carbone.
- Y est un atome d'hydrogène ou d'halogène, un radical alcoyle ou alcoxy de 1 à 4 atomes de carbone,
- n est un entier de 1 à 3 avec la possibilité, quand n est supérieur à 1, que les Y soient identiques ou différents.

Des composés préférés ont la formule I dans laquelle R est un ammonium substitué, un radical alcoyle de 1 à 8 atomes de carbone éventuellement substitué, plus particulièrement méthyle ou éthyle, un radical alcényle de 2 à 4 atomes de carbone, alcoxy carbonyl méthyle, phényle éventuellement susbtitué, phényle

(éventuellement substitué) carbonyl méthyle, ou un radical pyridinyl -méthyle.

La plupart de ces composés sont originaux.

Par contre les dérivés dans la formule desquels Y = H et R est un atome d'hydrogène, un méthyle ou éthyle sont connus, sans que cependant aient été décrites leurs propriétés fongicides.

Les composés utilisables selon l'invention peuvent être obtenus selon des procédés en soi connus.

Lorsque dans la formule I, le radical R est un radical alcoyle de 1 à 4 atomes de carbone, des produits peuvent être préparés selon un procédé en soi connu [cf Bull. Soc. Chim. Belg. 89 205 (1980)] (Procédé I) par action de chloroformiate d'alcoyle approprié sur le dérivé amido- acide des dérivés nicotiniques selon le schéma

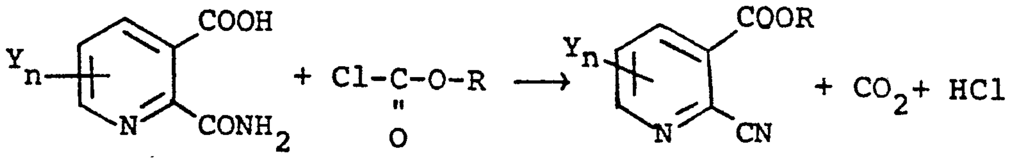

en milieu solvant, en présence d'un accepteur d'acide. Comme solvant, on peut citer par exemple des hydrocarbures aliphatiques (notamment le chlorure de méthylène) ou aromatiques éventuellement halogénés, des cétones, des nitriles. Comme accepteur d'acide, on peut citer une base minérale telle qu'un carbonate alcalin ou alcalino-terreux ou une base organique telle qu'une amine de basicité plus forte que celle du produit final notamment une amine tertiaire, par exemple la triéthylamine.

Les dérivés de formule I, dans laquelle R est un cation métallique M, peuvent être obtenus en particulier, lorsque M est un atome de métal alcalin et alcalino-terreux, par saponification de l'ester éthylique ou méthylique correspondant avec l'hydroxyde du métal considéré selon le schéma (Procédé II) :

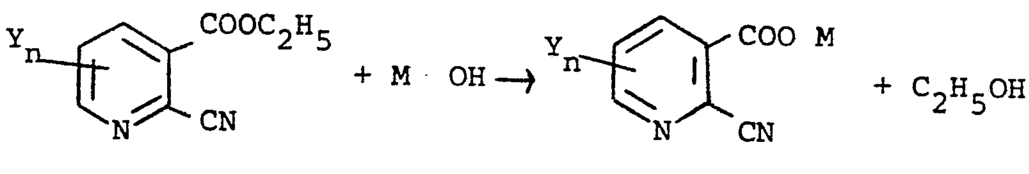

(II)

Les dérivés de formule I dans laquelle R n'est ni un alcoyle contenant un à quatre atomes de carbone, ni un cation métallique M, peuvent être obtenus selon plusieurs procédés (III) : d'abord on peut effectuer la réaction d'un composé de formule II avec un halogénure d'alcoyle approprié selon le schéma (Procédé III A) :

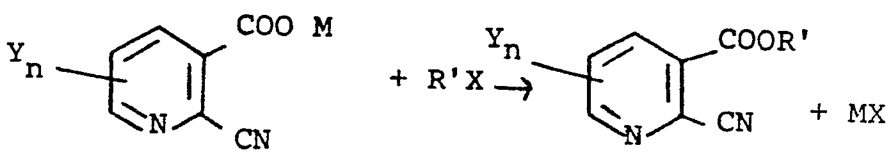

La réaction est effectuée en milieu solvant aliphatique ou aromatique comme par exemple les hydrocarbures éventuellement halogènes, (par exemple le toluène), ou des amides, cétones, ou nitriles (par exemple l'acétonitrile). Avantageusement, on opère en présence de catalyseur du type catalyseur de transfert de phase tels que par exemple la tris(dioxa-3,6 heptyl) amine (TDA-1) ou l'éther couronne 18-6.

On peut encore faire réagir un composé de formule II avec un agent chlorant pour donner un dérivé chlorure d'acide III que l'on fait réagir avec un alcool, selon le schéma (Procédé III B) :

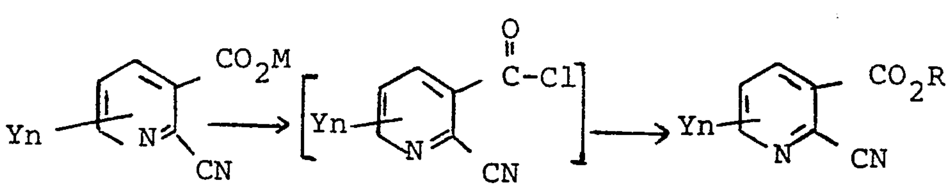

II III I

Le traitement de II par un agent chlorant, tel que phosgène, chlorure d'oxalyle ou chlorure de thionyle, dans un solvant aliphatique tel qu'un chloroalcane fournit le dérivé III. Celui-ci est transformé en composé I par réaction avec l'alcool approprié en présence de base organique telle que par exemple pyridine.

On peut encore effectuer une transestérification selon le schéma (Procédé III C) :

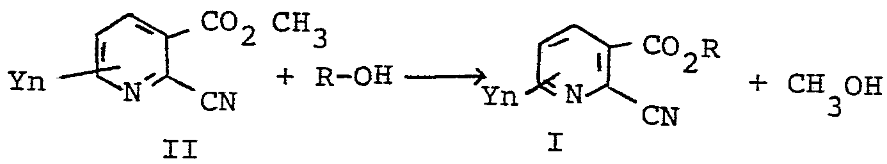

II I

La réaction est effectuée en solvant aromatique tel que par exemple le toluène, en présence d'une quantité catalytique d'une base comme le méthylate de sodium avec distillation de l'azéotrope méthanol-solvant au fur et à mesure de la formation du méthanol.

Les produits de formule I, dans laquelle R est un ammonium substitué peuvent être préparés par salification de l'acide correspondant (R = H) avec une amine appropriée [cf Chem. Pharm. Bull 27 2473 (1979)] (Procédé IV).

Les exemples suivants illustrent la préparation des composés selon l'invention et leurs propriétés fongicides. La structure de ces composés a été vérifiée par spectrographie RMN.

Exemple I : Cyano-2 nicotinate de potassium (composé 3)

On ajoute 20 g (0,113 moles) de cyano-2 nicotinate d'éthyle (composé 2) à une solution de 6 g (0,107 moles) d'hydroxyde de potassium dans 150 ml d'eau distillée. Le mélange est ensuite laissé à 25° C durant 10 heures, puis lavé par 2 fois 50 ml d'éther. La solution aqueuse est ensuite évaporée à sec pour fournir 19,1 g de (Composé 3) (Fus 300° C)

Exemple 2 : Cyano-2 nicotinate d'octyle (composé 6)

A 40 ml d'acétonitrile on ajoute 4,1 g (0,022 moles) du composé 3, 3,86 g (0,02 moles) de bromure d'octyle et 130 mg (0,0005 moles d'éther couronne 18-6. Le mélange réactionnel est porté à reflux durant 4 heures. Le solvant est ensuite évaporé, le résidu repris à l'eau puis extrait du dichlorométhane. Après séchage, évaporation puis purification sur colonne de silice, on obtient 3,9 g d'une huile jaune (75 %).

Exemple 3 : Cyano-2 nicotinate d'(éthoxy carbonyl) méthyle (composé 2)

40 ml de toluène contenant 4,1 g (0,022 moles) de (III), 3,35 g (0,02 moles) de bromoacétate d'éthyle, 0,3 ml de TDA-1 sont portés au reflux durant 4 heures. Le toluène est ensuite évaporé et le résidu repris à l'eau puis extrait au dichlorométhane. Après sèchage, évaporation et cristallisation dans le pentane, on obtient 4,7 g de cyano-2 nicotinate d'(éthoxy carbonyl) méthyle (Fus : 74° C).

Exemple 4 : Cyano-2 nicotinate de di-isopropyl ammonium (composé 31)

0,74 g (0,005 moles) d'acide cyano-2 nicotinique en suspension dans 40 ml d'acétone sont traités par 0,56 g (0,0056 moles) de di-isopropylamine. Le solvant est ensuite évaporé et le résidu cristallisé dans l'hexane : on obtient 1,24 g (99 %), Fus : 156,7° C de cyano-2 nicotinate de di-isopropyl ammonium.

Exemple 5 : cyano-2 nicotinate de (trichloro-2,2,2) éthyle (composé 63)

2,8 g (0.0022 moles) de chlorure d'oxalyle sont additionnés à une suspension de 4,1 g (0.022 mole) de composé 3 dans 50 ml de dichlorométhane. Le mélange est porté au reflux jusqu'à fin de dégagement gazeux, puis traité par une solution de 3,3 g (0.022 mole) de trichloroéthanol et de 3,5 g (0.044 mole) de pyridine dans 30 ml de dichlorométhane. Le mélange est alors traité à l'eau puis séché. Après évaporation du solvant et cristallisation dans le pentane, on obtient 4,55 g (78 %) de cyano-2 nicotinate de (trichloro-2,2,2) éthyle (Fus : 103° C).

Exemple 6 : Cyano-2 nicotinate de (pyridyl-2)-2 éthyle (composé 84)

Une solution de 8,1 g (0.05 mole) de cyano-2 nicotinate de méthyle et 7,4 g (0.06 mole) de pyridyl-2 éthanol dans 300 ml de toluène, est traitée par 0,3 g (0.005 mole) de méthylate de sodium. L'azéotrope méthanol-toluène est distillé puis le toluène est évaporé. Le résidu est traité à l'eau. Le précipité formé est filtré, séché puis lavé au pentane. On obtient 9,64 g (76 %) de composé 84 (Fus = 73.5° C).

Exemple 7 :

En opérant selon le mode opératoire aux exemples 2 et 6, on obtient les composés suivants dont la formule et les caractéristiques physico-chimiques sont consignées dans le tableau suivant :

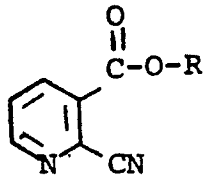

| | R | F°C | Procédé |
|---|---|---|---|
| 1 | $CH_3$ | 82,9 | I |
| 2 | $C_2H_5$ | 41 | II |
| 3 | K | 300 | II |
| 4 | iso$C_3H_7$ | 92 | III A |
| 5 | n$C_5H_{11}$ | huile | III A,B |
| 6 | n$C_8H_{17}$ | 25 (huile) | III A |
| 7 | $-CH_2-CH=CH_2$ | huile | III A |
| 8 | $-CH_2-CH=CH-Cl$ | 59,5 | III A |
| 9 | $CH_2-CH=CH-CH_3$ (E) | huile | III A |
| 10 | $CH_2-C\equiv CH$ | 112 | III A |
| 11 | 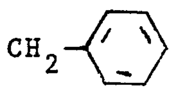 (benzyle) | 67 | III A |
| 12 | 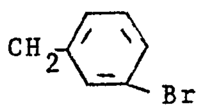 | 116 | III A |
| 13 | 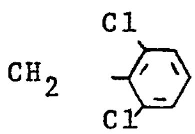 | 165 | III A |
| 14 | 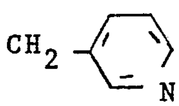 | 91 | III A |
| 15 | 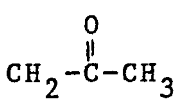 | 86 | III A |
| 16 | 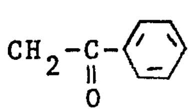 | 115 | III A |
| 17 | 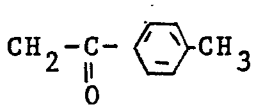 | 142 | III A |

| | R | F°C | Procédé |
|---|---|---|---|
| 18 | 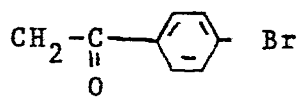 | 162 | III A |
| 19 | 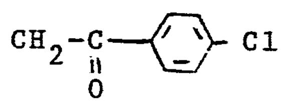 | 140,5 | III A |
| 20 | 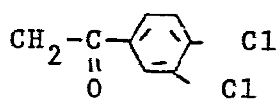 | 140 | III A |
| 21 | $CH_2-C(=O)-O\ CH_3$ | 116 | III A |
| 22 | $CH_2-C(=O)-OC_2H_5$ | 74 | III A |
| 23 | $-CH(CH_3)-C(=O)-OC_2H_5$ | 40 | III A |
| 24 | $-CH(C_2H_5)-C(=O)-OC_2H_5$ | 60 | III A |
| 25 | $-(CH_2)_3-C(=O)-OC_2H_5$ | 60,5 | III A |

| | R | F°C | Procédé |
|---|---|---|---|
| 26 | $-CH_2-CH=CH-C(=O)-OCH_3$ (E) | 93,5 | III A |
| 27 | $CH_2-CN$ | 64 | III A |
| 28 | $-CH_2-CH{\equiv}CH-CH_2-O$ 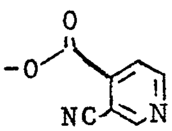 | 209 | III A |
| 29 | $-CH_2-$ 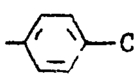 $-CH_2$ O 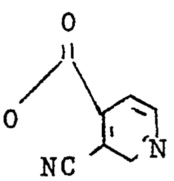 | 242 | III A |
| 30 | $HN^+(C_2H_5)_3$ | 65 | IV |
| 31 | $H_2N^+(CH(CH_3)_2)_2$ | 157 | IV |
| 32 | $H_2N^+(c-C_6H_{11})_2$ | 178 | IV |
| 33 | $H_2N^+(CH_2CH_2OH)_2$ | 115 | IV |
| 34 | $H_3N^+(CH_2)_{17}$ $CH_3$ | 79 | IV |
| 35 | H | 171 | 1 |

| | R | F°C | Procédé |
|---|---|---|---|
| 36 | $CH_2$ $CH_2$-O- 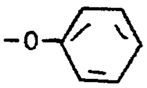 | 102 | III A |
| 37 | $CH_2CH_2$ 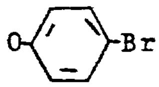 | 71 | III A |
| 38 | $CH_2CH_2Br$ | 65 | III A |
| 39 | $CH_2$ 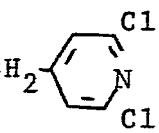 | 161,5 | III A |
| 40 | $CH_2$ 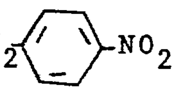 | 202 | III A |
| 41 | $CH_2$ 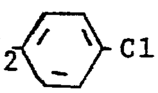 | 133 | III A |
| 42 | $CH_2$ 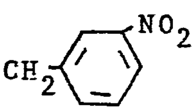 | 156,5 | III A |
| 43 | $CH_2$ 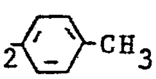 | 97,5 | III A |
| 44 | $CH_2$ 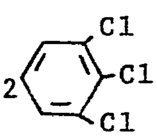 | 179 | III A |
| 45 | $CH_2CO_2$ $C(CH_3)_3$ | 47 | III A |

| | R | F°C | Procédé |
|---|---|---|---|
| 46 | $CH_3$ 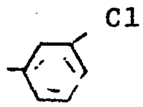 | 112 | III A |
| 47 | $CH_2$ 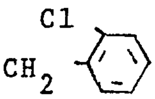 | 126 | III A |
| 48 | $CH_2$ 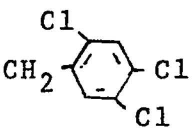 | 102 | III A |
| 49 | $CH_2$ 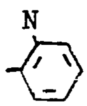 | 106.5 | III A, C |
| 50 | O<br>‖<br>$CH_2-C$ 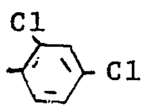 | 161.5 | III A |
| 51 | $C_4H_9$ | Huile | III A |
| 52 | CH2 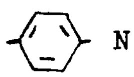 | 100 | III A |
| 53 | O<br>‖<br>$CH_2-C-OCH_2CH=CHCH_3$ | 123 | III A |
| 54 | $CH_3$<br>\|<br>$CH_2-Si-CH_2C_6H_5$<br>\|<br>$CH_3$ | Huile | III A |
| 55 | $CH_3$<br>\|<br>$CH_2-Si-CH_2$ 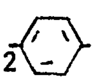 $C(CH_3)_3$<br>\|<br>$CH_3$ | Huile | III A |

| | R | F°C | Procédé |
|---|---|---|---|
| 56 | $CH(CO_2C_2H_5)_2$ | 93 | III A |
| 57 | 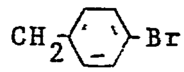 | 157 | III A |
| 58 | 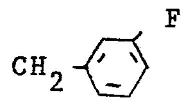 | 168 | III A |
| 59 | 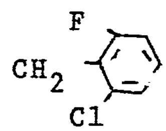 | 151 | III A |
| 60 | 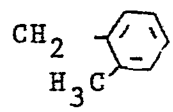 | 112 | III A |
| 61 | 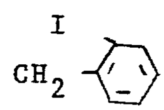 | 135 | III A |
| 62 | $CH_2-CF_3$ | 80,5 | III B |
| 63 | $CH_2$ $CCl_3$ | 103 | III B |
| 64 | $CH_2$ $CHCl_2$ | 83,5 | |
| 65 | 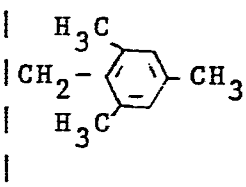 | 251 | III A |
| 66 | 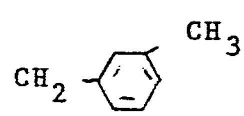 | 76 | III A |
| 67 | 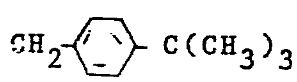 | 71 | III A |

| | R | F°C | Procédé |
|---|---|---|---|
| 68 | 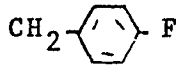 | 108 | III A |
| 69 | 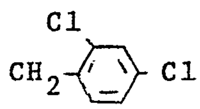 | 151 | III A |
| 70 | $C_2H_5$<br>$CH_2-CH-C_4H_9$ | Huile | III B |
| 71 | 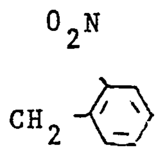 | 160 | III A |
| 72 | 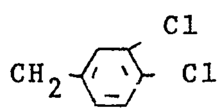 | 162 | III A |
| 73 | 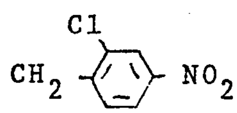 | 124 | III A |
| 74 | $(CH_2)_{15}CH_3$ | 108 | III B |
| 75 | 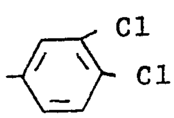 | 145,5 | III B |
| 76 | 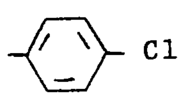 | 125 | III B |
| 77 | 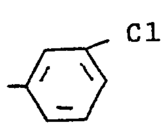 | 139,5 | III B |
| 78 | 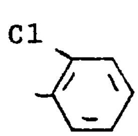 | 121 | III B |

| | R | F°C | Procédé |
|---|---|---|---|
| 79 | 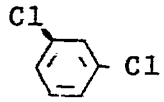 | 130 | III B |
| 80 | 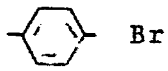 | 110,5 | III B |
| 81 | 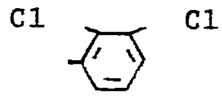 | 164,5 | III B |
| 82 | 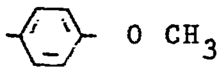 | 122 | III B |
| 83 | $(CH_2)_{11}$ $CH_3$ | 52 | IV B |
| 84 | $CH_2CH_2$ 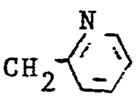 | 73,5 | III C |
| 85 | $CH_2$ 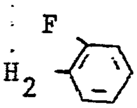 | 113 | III A |
| 86 | $CH_2CH_2-SC_2H_5$ | Huile | III B |
| 87 | $CH_2CH_2-I$ | 48 | III B |
| 88 | $C_3H_7$ | Huile | III B |
| 89 | $CH_2CH(CH_3)_2$ | Huile | III B |
| 90 | $H_2C$ 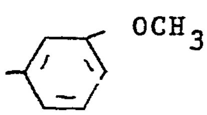 | 83,5 | III A |
| 91 | $CH_2CH_2Cl$ | 69 | III B |

| | R | F°C | Procédé |
|---|---|---|---|
| 92 | $C(CH_3)_3$ | 62 | III B |
| 93 | $CH_2$ 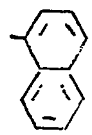 | 140 | III B |

Exemple 6 : Test de serre, sur Piricularia oryzae

Des plants de riz (variété Marcheti rosa) de hauteur 10 cm groupés en barquettes de 2, sont traités par arrosage au sol, avec une suspension aqueuse contenant :

- 30 mg du produit à tester
- 15 mg d'un agent tensioactif, condensat d'oxyde d'éthylène (20 moles) sur le monoléate de sorbitan
- eau qsp 30 ml

Cette suspension, appliquée à des pots carrés de 7 cm de côté, correspond à une dose d'environ 60 kg/ha de matière active. On laisse absorber le produit par le sol. Une partie des plants de riz n'a pas été traitée pour servir de témoins. Au bout de 24 heures après le traitement on contamine tous les plants de riz avec une suspension de spores de Piricularia oryzae obenues par grattage d'une culture in vitro, par pulvérisation sur les feuilles à raison de 5 ml de suspension par pot. On laisse incuber les pots pendant 48 h à 25°C sous 100° de'humidité relative .

On effectue l'observation au bout de 48 h après la contamination.

Dans ces conditions on observe qu'à la dose de 1000 ppm, une inhibition d'au moins 80 % du champignon est obtenue avec les composés 1 à 9, 11, 12, 14 à 18 et 20 à 27, 30 à 32, 35, 36, 38, 40, 42 à 45, 47 à 50, 52, 69 à 71, 73, 74, 77 à 82.

Cette évolution montre bien les propriétés fongicides systémiques des composés selon l'invention et leur action remarquable sur la piriculariose du riz.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... ainsi que d'autre matières actives connues à propriétés pesticides (notamment insecticides ou fongicides) ou à propriétés favorisant la croissance des plantes (notamment des engrais) ou à propriétés régulatrices de la croissance des plantes. Plus généralement les compsés selon l'invention peuvent être associés à tous les additifs solides ou liquides correspondant aux techniques habituelles de la mise en formulation.

Ces doses d'emploi dans le cas d'une utilisation comme fongicides des composés selon l'invention peuvent varier dans de larges limites, notamment selon la virulence des champignons et les conditions climatiques.

D'une manière générale des compositions contenant 0,5 à 5000 ppm de substance active conviennent bien ; ces valeurs sont indiquées pour les compositions prêtes à l'application. Ppm signifie "partie par million". La zone de 0,5 à 5000 ppm correspond à une zone de $5x10^{-5}$ à 0,5 % (pourcentage pondéraux).

En ce qui concerne les compositions adaptées au stockage et au transport, elles contiennent plus avantageusement de 0,5 à 95 % (en poids) de substance active.

Ainsi donc, les compositions à usage agricole selon l'invention peuvent contenir les matières actives selon l'invention dans de très larges limites, allant de $5.10^{-5}$% à 95 % (en poids).

Selon ce qui a déjà été dit les composés selon l'invention sont généralement associés à des supports et éventuellement des agents tensioactifs.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agricaulture, notamment sur la plante traitée. Le support peut être solide (argiles, silicates naturels ou synthétiques, silice,résines, cires, engrais solides, etc...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénosulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés. La présence d'au moins un agent tensioactif est généralement indispensable lorsque la matière active et/ou le support inerte ne sont pas

solubles dans l'eau et que l'agent vecteur de l'application est l'eau.

Les compositions utilisées dans l'invention peuvent être sous des formes assez diverses, solides ou liquides.

Comme formes de compositions solides, on peut citer les poudres pour poudrage (à teneur en matières actives pouvant aller jusqu'à 100 %).

Comme formes de compositions liquides ou destinées constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les granulés, les pâtes.

Les concentrés émulsionnables ou solubles comprennent le plus souvent 10 à 80 % de matière active, les émulsions ou solutions prêtes à l'application contenant, quant à elles, 0,001 à 20 % de matière active. En plus de la matière active et du solvant, les concentrés émulsionnables peuvent contenir, quand c'est nécessaire, un co-solvant approprié et de 2 à 20 % d'additifs appropriés, comme des stabilisants, des agents tensioactifs, notamment des émulsifs, des agents de pénétration, des inhibiteurs de corrosion, des colorants, des adhésifs.

A partir de ces concentrés, on peut obtenir par dilution avec de l'eau des émulsions de toute concentration désirée, qui conviennent particulièrement à l'application sur les cultures.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici la composition de plusieurs suspensions aqueuses selon l'invention :

Exemple A :

On prépare une suspension aqueuse comprenant :

- matière active (composé n° 4)............... 500 g/l
- agent mouillant (alcool synthétique en $C_{13}$ polyéthoxylé) ................................ 10 g/l
- agent dispersant (phosphate de polyaryl phénol éthoxylé salifié) ........................... 50 g/l
- antigel (propylèneglycol) .................. 100 g/l
- épaississant (polysacharide) ............... 1,6 g/l
- biocide (méthylhydroxy-4 benzoate sodé) .... 3,3 g/l
- eau ...................................Q.S.P.1 litre.

On obtient ainsi une suspension concentrée fluide.

Exemple B - Suspension aqueuse :

On prépare une suspension aqueuse comprenant :

- matière active (composé n° 7)............... 100 g/l
- agent mouillant (alkylphénol polyéthoxylé .. 5 g/l

- agent dispersant (Naphtalène sulfonate de Na 10 g/l
- antigel (Propylèneglycol) .................. 100 g/l
- épaississant (Polysacharide) ............... 3 g/l
- biocide (Formaldéhyde) ..................... 1 g/l
- eau ................................Q.S.P.1 litre.

Exemple C - Suspension aqueuse :

On prépare une suspension aqueuse comprenant :

- matière active (composé n° 22).............. 250 g/l
- agent mouillant (alcool synthétique en $C_{13}$ polyéthoxylé ............................. 10 g/l
- agent dispersant (lignosulfonate de solium) 15 g/l
- antigel (urée) ............................ 50 g/l
- épaississant (Polysacharide) ............... 2,5 g/l
- biocide (Formaldéhyde) ..................... 1 g/l
- eau ................................Q.S.P.1 litre.

Les poudres mouillables (ou poudre à pulvériser) sont habituellement préparées de manière qu'elles contiennent 20 à 95 % de matière active, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple, voici la composition de plusieurs poudres mouillables.

- matière active (composé n° 1 selon l'invention) 50 %
- alcool gras éthoxylé (agent mouillant)......... 2,5 %
- styrylphénol éthoxylé (agent dispersant)....... 5 %
- craie (support inerte) ........................42,5 %

Exemple E : Poudre mouillable à 10 %

- matière active (composé n° 4) .................10 %
- alcool synthétique oxo de type ramifié, en $C_{13}$ éthoxylé par 8 à 10 oxyde d'éthylène (agent mouillant) ...................................0,75 %

- lignosulfonate de calcium neutre (agent dispersant ............................................ 12 %
- carbonate de calcium (charge inerte) .......qsp 100 %

Exemple F : Poudre mouillable à 75 % contenant les mêmes ingrédients que dans l'exemple précédent, dans les proportions ci-après :

- matière active (composé n° 7) ................. 75 %
- agent mouillant ...................................1,50 %
- agent dispersant ..................................8 %
- carbonate de calcium (charge inerte) .......qsp 100 %

Exemple G : Poudre mouillable à 90 %

- matière active (composé n° 22) ..................90 %
- alcool gras éthoxylé (agent mouillant ........... 4 %
- styrylphénol éthoxylé (agent dispersant) ........ 6 %

Exemple H : Poudre mouillable à 50 %

- matière active (composé n° 4 selon l'invention)..50 %
- mélange de tensio-actifs anioniques et non ioniques (agent mouillant) .....................2,5 %
- lignosulfonate de sodium neutre (agent dispersant) ............................................ 5 %
- argile kaolinique (support inerte) .............42,5 %

Pour obtenir ces poudres à pulvériser ou poudres mouillables, on mélange intimement la matière active dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et cette suspension est utilisable très avantageusement en particulier pour l'application sur les feuilles des végétaux.

Les composés selon l'invention peuvent être avantageusement formulés sous la forme de granulés dispersibles dans l'eau également compris dans le cadre de l'invention.

Ces granulés dispersibles, de densité apparente généralement comprise entre environ 0,3 et 0,6, ont une dimension de particules généralement comprise entre environ 150 et 2000 et de préférence entre 300 et 1500 microns.

La teneur en matière active (composé n° 4) de ces granulés est généralement comprise entre environ 1 % et 90 %, et de préférence entre 25 % et 90 %.

Les reste du granulé est essentiellement composé d'une charge solide et éventuellement d'adjuvants tensio-actifs conférant au granulé des propriétés de dispersiblité dans l'eau. Ces granulés peuvent être essentiellement de deux types distincts selon que la charge retenue est soluble ou non dans l'eau. Lorsque la charge est hydrosoluble, elle peut être minérale et de préférence organique. On a obtenu d'excellents résultats avec l'urée. Dans le cas d'une charge insoluble, celle-ci est de préférence minérale, comme par exemple le kaolin ou la bentonite. Elle est alors accompagnée d'agents tensio-actifs (à raison de 2 à 20 % en poids du granulé), adjuvants tensio-actifs dont plus de la moitié est constituée par au moins un agent dispersant, essentiellement anionique, tel qu'un poly(naphtalène sulfonate alcalin ou alcalino terreux) ou un lignosulfonate alcalin ou alcalino-terreux, le reste étant constitué par des mouillants non ioniques ou anioniques tel qu'un alcoyl naphtalène sulfonate alcalin ou alcalin-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc..). On termine généralement par un concassage suivi d'un tamisage à la dimension de particule choisie dans les limites

mentionnées ci-dessus.

De préférence, il est obtenu par extrusion, en opérant comme indiqué dans les exemples ci-après.

Exemple I : Granulés dispersibles à 90 % de matière active

Dans un mélangeur, on mélange 90 % en poids de matière active (composé no 7) et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

Exemple J : Granulés dispersibles à 75 % de matière active

Dans un mélangeur, on mélange les constituants suivants :

- matière active (composé n° 22) .................. 75 %
- agent mouillant (alkylnaphtalène sulfonate de sodium ........................................ 2 %
- agent dispersant (polynaphtalène sulfonate de sodium) ........................................ 8 %
- charge inerte insoluble dans l'eau (kaolin) ..... 15 %

Ce mélange est granulé en lit fluide, en présence d'eau, puis séché, concassé et tamisé de manière à obtenir des granulés de dimension comprise entre 0,16 et 0,40 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

Comme cela a déjà été dit, les dispersions et émulsions aqueuses, par exemple des compositions obtenues en diluant à l'aide d'eau une poudre mouillable ou un concentré émulsionnable selon l'invention, sont comprises dans le cadre général des compositions utilisables dans la présente invention. Les émulsions peuvent être du type eau-dans-l'huile ou huile-dans-l'eau et elles peuvent avoir une consistance épaise comme celle d'une "mayonnaise".

L'invention concerne de plus un procédé de traitement des végétaux contre les maladies causées par les champignons phytopathogènes appartenant aux famille ples plus variées et notamment sur les Botrytis sp souches sensibles ou résistantes aux benzimidazoles, le piétin verse (souches sensibles ou résistantes eux benzimidazoles), les champignons des semenses tels que Pythium sp, Fusarium sp, Septoria sp, Rhizoctonia sp ou encore les champignons responsables des pourritures tels que Monilia sp, Penicillium sp, Rhizopus sp ou encore Venturia sp, Phytophthora sp. etc...

Ce procédé est caractérisé en ce qu'il consiste à appliquer sur ces végétaux une quantité efficace d'une composition contenant comme matière active un composé selon la formule (I). Par "quantité efficace" on entend une quantité suffisante pour permettre le contrôle et la destruction des champignons présents sur ces végétaux. Les doses d'utilisation peuvent toutefois varier dans de larges limites selon le champignon à combattre, le type de culture, les conditions climatiques, et selon le composé utilisé.

En pratique des doses allant de 1 g/hl à 500 g/hl correspondant sensiblement à des doses de matière active par hectare de 10 g/ha à 5000 g/ha environ donnent généralement de bons résultats.

**Revendications**

1) Dérivés nicotiniques de formule :

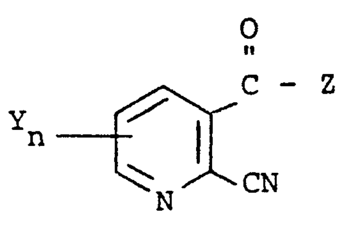

(I)

dans laquelle
- Z est un radical OR dans lequel
R est
• un atome d'hydrogène, un cation M/minéral, métallique ou ammonium, ou organique notamment ammonium substitué en particulier mono-, di-, ou trisubstitué par un radical alcoyle linéaire ou ramifié, de 1 à 18 atomes de carbone, cycloalcoyle de 3 à 6 atomes de carbone ou alcanol de 1 à 3 atomes de carbone, ou
• un radical alcoyle, linéaire ou ramifié, de 1 à 18 atomes de carbone, ou alcényle de 2 à 18 atomes de carbone, alcynyle de 2 à 5 atomes de carbone chacun de ces radicaux pouvant être substitué par au moins un substituant choisi dans le groupe comprenant :
- un atome d'halogène, un groupe cyano,
- un radical alcoxy ou alcoylthio de 1 à 4 atomes de carbone,
- un radical phénoxy éventuellement substitué par au moins un substituant choisi dans le groupe comprenant un atome d'halogène, un alcoyle de 1 à 4 atomes de carbone ou un alcoxy de 1 à 4 atomes de carbone,
- un groupe COOR'ou OCOR', dans lequel R' est un alcoyle de 1 à 4 atomes de carbone, un alcényle de 2 à 4 atomes de carbone ou un phényle chacun des radicaux alcoyle alcényle ou ou phényle étant éventuellement substitué par au moins un substituant choisi dans le groupe comprenant un atome d'halogène, un alcoyle de 1 à 4 atomes de carbone ou un alcoxy de 1 à 4 atomes de carbone,, un groupe I' de formule :

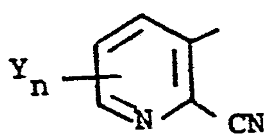

(I')

dans laquelle Y et n sont comme ci-après,
- ou un groupe phényle ou naphtyle éventuellement substitué par au moins un substituant choisi dans le groupe comprenant un atome d'halogène, un alcoyle de 1 à 4 atomes de carbone, un alcoxy de 1 à 4 atomes de carbone et le radical $NO_2$
- ou un groupe amino éventuellement substitué,
• ou un groupe phényle éventuellement substitué par au moins un substituant choisi dans le groupe comprenant un atome d'halogène, un alcoyle de 1 à 4 atomes de carbone et un alcoxy de 1 à 4 atomes de carbone
• ou pyridinyl alcoyl (de 1 à 3 atomes de carbone) éventuellement substitué par au moins 1 atome d'halogène et/ou un alcoyle de 1 à 4 atomes de carbone et/ou un groupe cyano,
• ou alcoyl- ou phényl- ou phénylalcoyl carbonylalcoyle, dont chaque partie alcoyle comprend 1 à 3 atomes de carbone et dont la partie phényle est éventuellement substituée par 1 à 5 substituants choisis dans le groupe comprenant un atome d'halogène, un alcoyle de 1 à 4 atomes de carbone, un alcoxy de 1 à 4 atomes de carbone et le radical $NO_2$
• ou un groupe tri alcoyl ($C_1$-$C_4$) silicyl alcoyle ($C_1$-$C_4$), la partie alcoyle pouvant être substituée par un phényle éventuellement substitué par un alcoyle de 1 à 4 atomes de carbone.
- Y est un atome d'hydrogène ou d'halogène, un radical alcoyle ou alcoxy
de 1 à 4 atomes de carbone,
- n est un entier de 1 à 3 avec la possiblité, quand n est supérieur à 1, que les Y soient identiques ou différents sous réserve que, lorsque Y est un atome d'hydrogène, R n'est pas un atome d'hydrogène ou un radical méthyle ou éthyle.

2) Dérivés selon la revendication 1 caractérisés en ce que, dans la formule I, R est un alcoyle de 1 à 8 atomes de carbone, un alcényle de 2 à 4 atomes de carbone.

3) Dérivés selon la revendication 1 caractérisés en ce que, dans la formule I, Y est un atome d'hydrogène, R est radical alcoyle de 3 à 8 atomes de carbone, un radical alcényle de 2 à 4 atomes de carbone, alcoxy carbonyl méthyle, phényle éventuellement substitué, phényl (éventuellement substitué) carbonyl méthyle, ou un radical pyridinyl -méthyle.

4) Procédé de préparation des composés selon l'une des revendications 1 à 3, dans la formule desquels R n'est autre qu'un atome de métal caractérisé en ce qu'on effectue la réaction selon le schéma

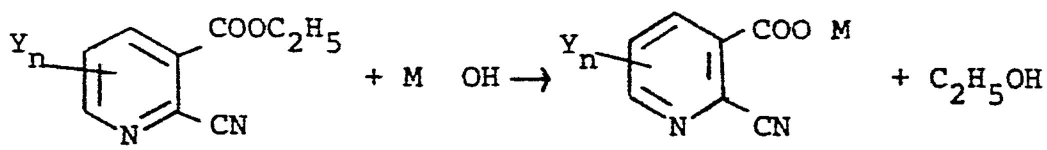

dans lequel M est un atome de métal alcalin ou alcalino-terreux, éventuellement en présence d'un solvant.

5) Procédé de préparation des composés selon les revendications 1 à 3 dans la formule desquels M est

un atome de métal alcalin ou alcalino-terreux, caractérisé en ce qu'on effectue la réaction selon le schéma :

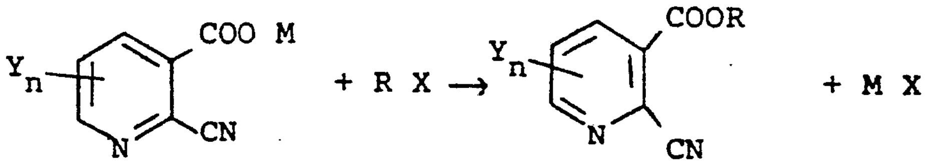


6) Procédé de préparation des composés selon la revendication 1 caractérisé en ce qu'on fait réagir un composé de formule II avec un agent chlorant pour donner un dérivé chlorure d'acide III que l'on fait réagir avec un alcool selon le schéma :

a)

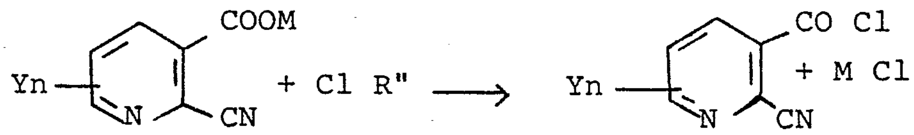


II III

dans un solvant notamment aliphatique

b) On fait réagir le composé III avec l'alcool approprié en présence d'une base organique telle que la pyridine, selon le schéma :

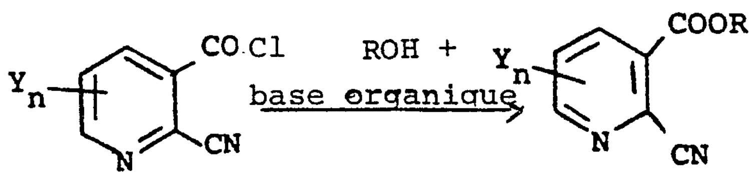


7) Procédé de préparation de composés selon la revendication 1, caractérisé en ce qu'on fait réagir un cyano-2 nicotinate de méthyle avec un alcool en milieu solvant notamment aromatique en présence d'une quantité catalytique d'un alcoolate alcalin selon la schéma :

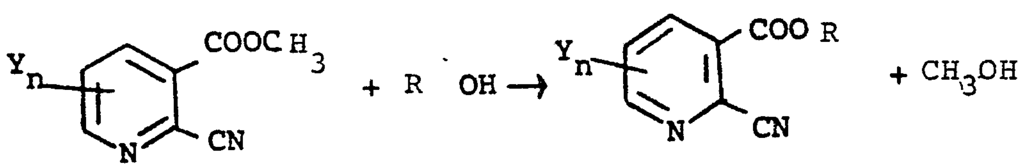


avec distillation azéotropique du méthanol au fur et à mesure de sa formation.

8) Procédé de préparation des composés selon la revendication 1 dans laquelle R est un cation ammonium substitué caractérisé en ce qu'on salifie l'acide correspondant (avec R = H) avec une amine appropriée.

9) Compositions fongicides caractérisées en ce qu'elles contiennent, comme matière active, au moins un composé de formule :

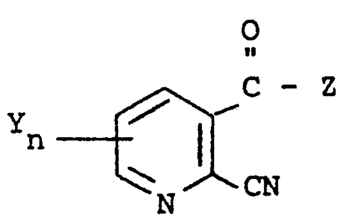

(I)

dans laquelle Z est soit un radical OR dans lequel R est

• un atome d'hydrogène, un cation M/minéral, métallique ou ammonium, ou organique notamment ammonium substitué en particulier mono-, di-, ou trisubstitué par un radical alcoyle linéaire ou ramifié, de 1 à 18 atomes de carbone, cycloalcoyle de 3 à 6 atomes de carbone ou alcanol de 1 à 3 atomes de

carbone, ou

• un radical alcoyle, linéaire ou ramifié, de 1 à 18 atomes de carbone, ou alcényle de 2 à 18 atomes de carbone, alcynyle de 2 à 5 atomes de carbone chacun de ces radicaux pouvant être substitué par au moins un substituant choisi dans le groupe comprenant un atome d'halogène, un groupe cyano, un radical alcoxy ou alcoylthio de 1 à 4 atomes de carbone, un radical phénoxy éventuellement substitué par au moins un substituant choisi dans le groupe comprenant un atome d'halogène, un alcoyle de 1 à 4 atomes de carbone ou un alcoxy de 1 à 4 atomes de carbone, un groupe COOR' ou OCOR', dans lequel R' est un alcoyle de 1 à 4 atomes de carbone, un alcényle de 2 à 4 atomes de carbone ou phényle éventuellement substitué par au moins un substituant choisi dans le groupe comprenant un atome d'halogène, un alcoyle de 1 à 4 atomes de carbone ou un alcoxy de 1 à 4 atomes de carbone,, un groupe I' de formule

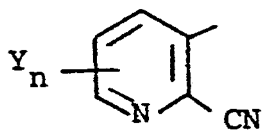

I'

dans laquelle Y et n sont comme ci-après,

• ou un groupe phényle éventuellement substitué par au moins 1 substituant choisi dans le groupe comprenant un atome d'halogène, un alcoyle de 1 à 4 atomes de carbone et un alcoxy de 1 à 4 atomes de carbone,

• ou un groupe amino éventuellement substitué,

• ou pyridinyl alcoyl (de 1 à 3 atomes de carbone) éventuellement substitué par au moins 1 atome d'halogène ou un alcoyl de 1 à 4 atomes de carbone ou un groupe cyano,

• ou alcoyl- ou phényl- ou alcoylphényl carbonyle dont la partie alcoyle comprend 1 à 3 atomes de carbone et dont la partie phényle est éventuellement substitué par 1 à 5 substituants choisis dans le groupe comprenant un atome d'halogène, un alcoyle de 1 à 4 atomes de carbone, un alcoxy de 1 à 4 atomes de carbone et le radical $NO_2$

• ou un groupe tri alcoyl ($C_1$-$C_4$) silicium la partie alcoyle pouvant être substitué par un phényle éventuellement substitué par 1 alcoyle de 1 à 4 atomes de carbone.

- Y est un atome d'hydrogène ou d'halogène, un radical alcoyle ou alcoxy
de 1 à 4 atomes de carbone,

- n est un entier de 1 à 3 avec la possibilité, quand n est supérieur à 1 que les Y soient identiques ou différents.

10) Composition selon la revendication 9 caractérisée en ce que dans la formule, R est un radical alcoyle de 1 à 8 atomes de carbone.

11) Composition selon la revendication 7 caractérisée en ce que dans la formule, R est un radical méthyle ou éthyle.

12)Procédé de traitement des plantes contre les maladies fongiques caractérisé en ce qu'on utilise des compositions selon l'une des revendications 9 à 11.